# EUROPEAN PATENT SPECIFICATION

(11) **EP 4 527 372 B1**
(45) Date of publication and mention of the grant of the patent: **25.11.2026**
(21) Application number: 23854436.5
(22) Date of filing: 15.08.2023
(51) Int. Cl.: A61K 9/00, A61K 9/14, A61K 9/16, A61K 47/34, A61K 31/445, A61K 31/55, A61K 31/58, A61K 31/485, A61K 38/00

(54) **PREPARATION METHOD FOR LONG-ACTING SUSTAINED RELEASE IMPLANT**
HERSTELLUNGSVERFAHREN FÜR IMPLANTAT MIT VERZÖGERTER FREISETZUNG UND LANGZEITWIRKUNG
PROCÉDÉ DE PRÉPARATION D'UN IMPLANT À LIBÉRATION PROLONGÉE À ACTION LONGUE

(30) Priority: 15.08.2022 CN 202210981508
(43) Date of publication of application: 26.03.2025
(73) Proprietor: Shenzhen Sciencare Medical Industries Co.,Ltd, Shenzhen, Guangdong 518000 (CN)
(72) Inventor: LIU, Daichun, Shenzhen, Guangdong 518000 (CN); QU, Wei, Shenzhen, Guangdong 518000 (CN); YAN, Xieguo, Shenzhen, Guangdong 518000 (CN); YIN, Shugui, Shenzhen, Guangdong 518000 (CN); ZHANG, Tao, Shenzhen, Guangdong 518000 (CN); CHEN, Zeqin, Shenzhen, Guangdong 518000 (CN); FU, Xiaofang, Shenzhen, Guangdong 518000 (CN); LUO, Jingling, Shenzhen, Guangdong 518000 (CN); ZHANG, Xuan, Shenzhen, Guangdong 518000 (CN); QIU, Xinmin, Shenzhen, Guangdong 518000 (CN)
(74) Representative: karo IP
(86) International application number: PCT/CN2023/113096
(87) International publication number: WO 2024/037535

(56) References cited:
- WO-A1-2021/053171
- CN-A- 1 658 836
- CN-A- 101 404 979
- CN-A- 102 271 725
- CN-A- 105 310 974
- CN-A- 105 560 161
- CN-A- 105 560 161
- CN-A- 108 969 468
- CN-A- 114 340 598
- US-A1- 2010 047 306
- ANONYMOUS: "Cryogenic grinding", WIKIPEDIA, 24 April 2011 (2011-04-24), XP093303082, Retrieved from the Internet <URL:https://en.wikipedia.org/wiki/Cryogenic_grinding>
- HIROAKI OKADA ET AL: "BIODEGRADABLE MICROSPHERES IN DRUG DELIVERY", CRITICAL REVIEWS IN THERAPEUTIC DRUG CARRIER SYSTEMS, BEGELL HOUSE PUBLISHING INC, US, vol. 12, no. 1, 1 January 1995 (1995-01-01), pages 1 - 99, XP000604141, ISSN: 0743-4863

## Description

### Cross-reference to Related Applications

The present disclosure claims priority to Chinese Patent Application No. 202210981508.9, entitled "preparation method of long-acting sustained and controlled release implant", filed to China National Intellectual Property Administration on August 15, 2022.

### Field of the Invention

The present disclosure relates to the field of pharmaceutical formulations, and particularly to a preparation method of a long-acting sustained and controlled release implant.

### Background of the Invention

Long-acting sustained and controlled release injections are typically administered by subcutaneous or intramuscular injection, creating a depot at the injection site to achieve a long-acting release effect. The depot provides sustained drug release over a period ranging from several days to several months, with the advantages of reduced administration frequency, simplified drug treatment regimens, decreased pharmacokinetic fluctuations, and fewer adverse reactions. However, there are some limitations in its application, such as the need for medical treatment, potential dose burst release, and variability between different formulations. As a result of these clinical or pharmaceutical development challenges, only over 20 long-acting sustained and controlled release injection chemicals have been marketed over the last 30 years, mainly used in the fields of antipsychotics, hormone therapy, and drug rehabilitation. According to the release mechanism, they can be roughly divided into three categories: (1) sustained release of active pharmaceutical ingredients, where the active pharmaceutical ingredients generally have poor water solubility, and a sustained release effect is achieved through the slow dissolution and release of the active pharmaceutical ingredients. (2) Drug self-aggregation sustained release, where the active pharmaceutical ingredients aggregate in the body to form a gelatinous depot to achieve a long-acting release effect. (3) Sustained release of excipients, where the excipients slowly and continuously diffuse or degrade at the administration site, thereby achieving a long-acting release of the active pharmaceutical ingredients. For the first two release mechanisms, nano/microcrystal suspension injection or in situ hydrogel injection is mainly adopted, while for the third release mechanism, implants are produced mainly by microspheres, in situ hydrogels, or hot melt extrusion (HME).

HME, also known as melt extrusion, refers to a technology in which a drug and a carrier excipient are uniformly mixed in a molten state and extruded at a certain pressure, speed, and shape. This technology enables the drug to be dispersed in the carrier in molecular, amorphous, or metastable form, which may enhance the dissolution rate of the poorly soluble drug and improve its bioavailability. It is a rapidly developing new formulation technology that has shown remarkable advantages in the preparation of sustained and controlled release formulations and drug delivery systems. Additionally, it is cost-effective, has a simple process, good reproducibility, and high production efficiency, offering broad development prospects in the field of pharmaceutical research and development.

Thermoplastic sustained and controlled release polymers and water-soluble drugs are prepared into implants through HME, and the drug particles are uniformly distributed in the polymer matrix. During extrusion, the molten drug-loaded material coats the drug particles. Thus, the size and surface of the pore structures formed are primarily a function of the size and surface area of the drug particles. The polymer matrix includes closed and open pore structures. In the closed pore structure, the drug particles are completely encapsulated/closed by the polymer matrix, while in the open pore structure, the drug particles are exposed to the fluid medium in which the implant is placed. Thus, during the initial phase of implant administration, drug particles exposed at the outer surface and in the open pore structure are rapidly released, resulting in a significant dose burst release effect. This burst release phenomenon is more obvious for drugs with good water solubility, which may bring certain safety risks to patients. In the present disclosure, a new preparation method of a long-acting implant is disclosed, i.e., adopting a coating or thermal passivation process, which can control the burst release of the drug at the initial stage of administration, maintain the steady release of the drug, and achieve the goal of zero-order or first-order drug release. Drugs include varenicline, buprenorphine, donepezil, triamcinolone acetonide, octreotide, leuprorelin, goserelin, exenatide, or pharmaceutically acceptable salts thereof.

According to CN111714442, an active pharmaceutical ingredient is mixed with (poly(lactic-co-glycolic acid) and then dissolved in a first solvent for HME to obtain a first extrudate. The first extrudate is immersed in a second solvent to obtain the implant. In this method, the organic solvent is used for many times, an intermediate product needs to be dried, the process is complicated, and the risk of organic solvent residue is high.

According to US20190350844, a long-acting depot composition, such as microsphere, implant, oily solution, liposome, suspension, microemulsion, and in situ hydrogel, including varenicline or pharmaceutically acceptable derivatives thereof and one or more biodegradable or non-biodegradable carriers is provided, which can effectively prevent patients from stopping treatment on their own through subcutaneous/intramuscular injection or implantation. Administration regimens ranging from once every three days to once every six months may reduce administration frequency, improve patient compliance, and reduce the risk of adverse reactions. Since varenicline belongs to BCS class I and has good water solubility, the above dosage forms usually have the burst release phenomenon after administration, bringing certain safety risks to patients.

According to CN102755627, acetic acid and ethanol are mixed to prepare a mixed solvent, and then a pharmaceutically acceptable carrier and goserelin are dissolved in the mixed solvent and freeze-dried to remove the solvent to prepare particles or fine powders. Then, the particles or fine powders are placed in a hot melt extruder, extruded into strips, and cut into cylinders. In this technology, a large amount of organic solvent is used, and the process of removing the organic solvent by freeze drying has a long production cycle and a large energy consumption.

According to CN106580868, a water-insoluble/micro-soluble drug and a release modifier are dissolved in an organic solvent, a water-insoluble polymer is added, and then the organic solvent is removed to obtain a solid mixture. The solid mixture is placed in a hot melt extruder, extruded, and cut to obtain an implant. The addition of the release modifier to the prescription may adjust the drug release rate to some extent. However, for the implant, only a few release modifiers are available for injection, and the more complex the composition of the prescription, the higher the risk of injection. Furthermore, a large amount of organic solvent is used for dissolving the drug and release modifier, and it is difficult to control the residual organic solvent in the implant.

According to WO 2021/053171 A1, an extruded depot form is provided for sustained active substance release, comprising at least one active substance and at least one first compound of the class of biodegradable organic polymers on the basis of lactic acid and/or glycolic acid.

In view of the problems existing in the related art, it is necessary to find a preparation method of a long-acting sustained and controlled release implant which is safe, has low energy consumption and a short cycle, and is easy to control.

### Summary of the Invention

In view of the problems existing in the related art, the present disclosure provides a preparation method of a long-acting sustained and controlled release implant. The preparation method is safe, energy-efficient, has a short cycle time, and is easy to control. It can prevent burst release while ensuring sustained release of the implant.

In order to achieve the above-mentioned object, the present disclosure adopts the following technical solutions.

The present invention provides a preparation method of an implant, including the steps of:
(1) mixing an active drug with a biodegradable polymer, freeze-grinding, drying, and sieving, resulting in a mixture;
(2) performing melt extrusion on the mixture obtained in step (1), then cooling, shaping, and pelletizing to obtain shaped solids; and
(3) performing thermal passivation or coating on the shaped solids obtained in step (2), then drying to obtain an implant;
wherein the thermal passivation in step (3) is performed at a temperature of 90-140°C for 5-30 min.

Further, a weight ratio of the active drug to the biodegradable polymer is 30-65: 35-70, preferably 35-60: 40-65.

Further, the active drug includes varenicline, buprenorphine, donepezil, triamcinolone acetonide, octreotide, leuprorelin, goserelin, exenatide, or pharmaceutically acceptable salts thereof.

Further, the biodegradable polymer includes one or more of polylactide (PLA), polyglycolic acid (PGA), poly(lactide-co-glycolide) (PLGA), polycaprolactone (PCL), PLA-PEG, PLGA-PEG, PLGA-PEG-PLGA, PLA-PEG-PLA, and PCL-PEG-PCL, preferably PLA, PLGA, PLA-PEG, PLGA-PEG, PLGA-PEG-PLGA, and PLA-PEG-PLA, and further preferably PLA and/or PLGA.

Further, molecular chains of PLA and PLGA carry anionic or cationic groups or do not carry anionic or cationic groups. More preferably, end groups of PLA and PLGA are alkyl ester groups or carboxyl groups. More preferably, when the biodegradable polymer is at least one of PLA and PLGA, a molar ratio of lactide to glycolide is 100: 0 to 50: 50. More preferably, when the biodegradable polymer is at least one of PLA and PLGA, the molar ratio of lactide to glycolide is 100: 0 to 75: 25.

Further, the biodegradable polymer of the present disclosure may be biodegraded into carbon dioxide and water in vivo, has good biocompatibility, and may be a single polymer or a mixture of multiple polymers. For example, the mixture may include a combination of PLGAs, in which lactide and glycolide have the same molar ratio and molecular weight but different end groups; a combination of PLGAs, in which lactide and glycolide have the same molar ratio and end group but different molecular weights; a combination of PLGAs, in which lactide and glycolide have the same molecular weight and end group but different molar ratios; and a combination of PLGAs, in which lactide and glycolide have different molar ratios, end groups, and molecular weights.

Further, the freeze-grinding in step (1) is performed at a temperature of -70°C to -20°C, preferably -50°C to -25°C.

A particle size of the frozen ground mixture in step (1) is not more than 20 mesh, preferably not more than 40 mesh, and further preferably not more than 60 mesh.

The thermal passivation in step (3) is performed at a temperature of 70-150°C for 2-60 min, preferably 90-140°C for 5-30 min, and further preferably 95-130°C for 8-30 min.

Further, a coating solution used for the coating in step (3) includes a sustained-release composition and an organic solvent; the sustained-release composition includes one or more of PLA, PGA, PLGA, PLA-PEG, PLGA-PEG, PLGA-PEG-PLGA, PLA-PEG-PLA, and preferably PLA and/or PLGA.

Further, a weight proportion of the sustained-release composition in the coating solution is 3-30%, preferably 6-25%.

Further, each of PLA and PLGA has a weight-average molecular weight (Mw) of 7,000-150,000 Da and an intrinsic viscosity of 0.1-2.5 dL/g, and preferably an Mw of 9,000-120,000 Da and an intrinsic viscosity of 0.2-1.2 dL/g.

Further, the organic solvent includes one or more of dimethyl sulfoxide, methanol, acetone, acetonitrile, dichloromethane, trichloromethane, tetrahydrofuran, ethyl acetate, and preferably ethyl acetate and/or dichloromethane.

Further, the drying in step (3) is specifically air drying at room temperature for 2-4 days, or vacuum drying at 30-45°C for 18-72 hours. Preferably, the drying is vacuum drying at 35-40°C for 24-48 hours.

Further, the implant prepared by the above-mentioned preparation method may have the shape of a granule, a cylinder, or a fine rod, and preferably the shape of a cylinder or a fine rod.

Further, when the implant has the shape of a cylinder or a fine rod, it has a length of not more than 6 cm and a diameter of not more than 3 mm. Preferably, when the implant has the shape of a cylinder or a fine rod, it has a length of not more than 5 cm and a diameter of not more than 2.5 mm. More preferably, when the implant has the shape of a cylinder or a fine rod, it has a length of not more than 3.5 cm and a diameter of not more than 2.0 mm.

Further, when the implant has the shape of a cylinder or a fine rod, it has an aspect ratio of (10-50): 1. Preferably, when the implant has the shape of a cylinder or a fine rod, it has an aspect ratio of (15-40): 1. More preferably, when the implant has the shape of a cylinder or a fine rod, it has an aspect ratio of (20-30): 1.

Technical effects achieved by the present disclosure are as follows.
1. According to the method of the present disclosure, a protective film can be formed on the outer surface of the implant by performing thermal passivation on the implant of water-soluble drugs, especially the BCS class 1 and BCS class 3 drugs, including some polypeptide drugs with acceptable thermal stability, so as to block the rapid release of drug particles attached to the outer surface of the implant, thereby controlling the burst release phenomenon, reducing the fluctuation of the drug in the blood, and improving the safety of the long-acting sustained and controlled release formulation.
2. According to the present disclosure, the melt extrusion and coating/passivation methods are adopted to achieve a dual sustained and controlled release mechanism combining matrix-type and film-coating control. Unlike dual film coating sustained and controlled release, the synergistic effect of matrix-type and film coating control enables the implant to ensure sustained release while preventing burst release.

### Brief Description of the Drawings

Fig. 1 is a scanning electron microscope (SEM) view of the morphology of a sample of comparative example 1;
Fig. 2 is a SEM view of the morphology of a sample of example 1;
Fig. 3 is a SEM view of the morphology of a sample of example 2;
Fig. 4 is a SEM view of the morphology of a sample of example 3;
Fig. 5 is a SEM view of the morphology of a sample of comparative example 2;
Fig. 6 is a SEM view of the morphology of a sample of example 4;
Fig. 7 is a SEM view of the morphology of a sample of example 5;
Fig. 8 is a SEM view of the morphology of a sample of example 6;
Fig. 9 is a SEM view of the morphology of a sample of comparative example 8;
Fig. 10 is a test view of in vitro release degrees of samples of the examples; and
Fig. 11 is a test view of in vitro release degrees of samples of the comparative example.

### Detailed Description of the Embodiments

Implementations of the present disclosure are illustrated below through the specific examples. Other advantages and efficacies of the present disclosure may be readilv annreciated bv a person skilled in the art from what is disclosed in this specification.

Before specific implementations of the present disclosure are described in further detail, it is to be understood that the scope of the present disclosure is not limited to the particular implementation solutions described below. It is also to be understood that the terminology used in the examples of the present disclosure is for the purpose of describing particular implementation solutions, and is not intended to limit the scope of the present disclosure.

When the examples give ranges of values, it is to be understood that, unless otherwise stated in the present claims, the two endpoints of each range of values, and any of the values between the two endpoints, are optional. Unless defined otherwise, all technical and scientific terminologies used herein have the same meaning as commonly understood by a person skilled in the art to which the present disclosure belongs.

It should be noted that the ingredients used in the present disclosure are all common commercially available products, and therefore the source thereof is not specifically limited.

### Example 1:

In this example, components of active pharmaceutical ingredient and excipients for the preparation of an implant were: 30% (W/W) of varenicline tartrate and 70% (W/W) of polylactic acid (PLA). The polylactic acid (PLA) had an Mw of 20 kDa, an intrinsic viscosity of 0.20 dL/g, and an alkyl ester group as the end group.
1) 9.0 g of varenicline tartrate and 21.0 g of polylactic acid (PLA) were mixed, frozen ground at -40°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 95°C, screw speed at 100 RPM, and outlet pressure below 40 Bar) and then pelletized to obtain a cylindrical implant with a diameter of 1.6 mm.
3) The implant obtained in step 2) was subjected to thermal passivation at 95°C for 30 min and then cooled to room temperature to obtain an implant with a diameter of 1.6 mm and an aspect ratio of 30.

### Example 2:

In this example, components of active pharmaceutical ingredient and excipients for the preparation of an implant were: 30% (W/W) of varenicline tartrate and 70% (W/W) of polylactic acid (PLA). The polylactic acid (PLA) had an Mw of 20 kDa, an intrinsic viscosity of 0.20 dL/g, and an alkyl ester group as the end group.
1) 9.0 g of varenicline tartrate and 21.0 g of polylactic acid (PLA) were mixed, frozen ground at -40°C, the moisture content being controlled to 3.7%, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 95°C, screw speed at 100 RPM, and outlet pressure below 40 Bar) and then pelletized to obtain a cylindrical implant with a diameter of 1.6 mm.
3) The implant obtained in step 2) was subjected to thermal passivation at 110°C for 20 min and then cooled to room temperature to obtain an implant with a diameter of 1.6 mm and an aspect ratio of 30.

### Example 3

In this example, components of active pharmaceutical ingredient and excipients for the preparation of an implant were: 30% (W/W) of varenicline tartrate and 70% (W/W) of polylactic acid (PLA). The polylactic acid (PLA) had an Mw of 20 kDa, an intrinsic viscosity of 0.20 dL/g, and an alkyl ester group as the end group.
1) 9.0 g of varenicline tartrate and 21.0 g of polylactic acid (PLA) were mixed, frozen ground at -40°C, the moisture content being controlled to 3.7%, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 95°C, screw speed at 100 RPM, and outlet pressure below 40 Bar) and then pelletized to obtain a cylindrical implant with a diameter of 1.6 mm.
3) The implant obtained in step 2) was subjected to thermal passivation at 120°C for 10 min and then cooled to room temperature to obtain an implant with a diameter of 1.6 mm and an aspect ratio of 30.

### Example 4:

In this example, components of active pharmaceutical ingredient and excipients for the preparation of an implant were: 50% (W/W) of buprenorphine hydrochloride and 50% (W/W) of PLGA. The PLGA had an Mw of 100 kDa, an intrinsic viscosity of 0.82 dL/g, a molar ratio of components of 75: 25, and a carboxyl group as the end group.
1) 10.0 g of buprenorphine hydrochloride and 10.0 g of PLGA were mixed, frozen ground at -60°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 125°C, screw speed at 150 RPM, and outlet pressure below 50 Bar) and then pelletized to obtain a cylindrical implant with a diameter of 3.0 mm.
3) The implant obtained in step 2) was coated. A coating solution was 6% polylactic acid (PLA had an Mw of 120 kDa and a carboxyl group as the end group), and an organic solvent was ethyl acetate. The coated implant was vacuum-dried at 40°C for 24 hours to obtain an implant with a diameter of 3.0 mm and an aspect ratio of 25.

### Example 5:

In this example, components of active pharmaceutical ingredient and excipients for the preparation of an implant were: 50% (W/W) of buprenorphine hydrochloride and 50% (W/W) of PLGA. The PLGA had an Mw of 100 kDa, an intrinsic viscosity of 0.82 dL/g, a molar ratio of components of 75: 25, and a carboxyl group as the end group.
1) 10.0 g of buprenorphine hydrochloride and 10.0 g of PLGA were mixed, frozen ground at -60°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 125°C, screw speed at 150 RPM, and outlet pressure below 50 Bar) and then pelletized to obtain a cylindrical implant with a diameter of 3.0 mm.
3) The implant obtained in step 2) was coated. A coating solution was 10% polylactic acid (PLA had an Mw of 120 kDa and a carboxyl group as the end group), and an organic solvent was ethyl acetate. The coated implant was vacuum-dried at 40°C for 24 hours to obtain an implant with a diameter of 3.0 mm and an aspect ratio of 25.

### Example 6:

In this example, components of active pharmaceutical ingredient and excipients for the preparation of an implant were: 50% (W/W) of buprenorphine hydrochloride and 50% (W/W) of PLGA. The PLGA had an Mw of 100 kDa, an intrinsic viscosity of 0.82 dL/g, a molar ratio of components of 75: 25, and a carboxyl group as the end group.
1) 10.0 g of buprenorphine hydrochloride and 10.0 g of PLGA were mixed, frozen ground at -60°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 125°C, screw speed at 150 RPM, and outlet pressure below 50 Bar) and then pelletized to obtain a cylindrical implant with a diameter of 3.0 mm.
3) The implant obtained in step 2) was coated. A coating solution was 25% polylactic acid (PLA had an Mw of 120 kDa and a carboxyl group as the end group), and an organic solvent was ethyl acetate. The coated implant was vacuum-dried at 40°C for 24 hours to obtain an implant with a diameter of 3.0 mm and an aspect ratio of 25.

### Example 7:

In this example, components of active pharmaceutical ingredient and excipients for the preparation of an implant were: 60% (W/W) of donepezil and 40% (W/W) of PLGA. The PLGA had an Mw of 40 kDa, an intrinsic viscosity of 0.32 dL/g, a molar ratio of 85: 15, and a carboxyl group as the end group.
1) 24.0 g of donepezil and 16.0 g of PLGA were mixed, frozen ground at -30°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 120°C, screw speed at 120 RPM, and outlet pressure below 30 Bar) and then pelletized to obtain a cylindrical implant with a diameter of 2.0 mm.
3) The implant obtained in step 2) was subjected to thermal passivation at 125°C for 25 min and then cooled to room temperature to obtain an implant with a diameter of 2.0 mm and an aspect ratio of 24.

### Example 8:

In this example, components of active pharmaceutical ingredient and excipients for the preparation of an implant were: 65% (W/W) of triamcinolone acetonide hydrochloride and 35% (W/W) of PLA-PEG-PLA. PLA-PEG-PLA had an Mw of 12 kDa, a mass percentage of PEG of 5%, and an intrinsic viscosity of 0.12 dL/g.
1) 26.0 g of triamcinolone acetonide hydrochloride and 14.0 g of PLA-PEG-PLA were mixed, frozen ground at -40°C, the moisture content being controlled to 5.0%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 90°C, screw speed at 80 RPM, and outlet pressure below 30 Bar) and then pelletized to obtain a cylindrical implant with a diameter of 2.5 mm.
3) The implant obtained in step 2) was subjected to thermal passivation at 90°C for 20 min and then cooled to room temperature to obtain an implant with a diameter of 2.5 mm and an aspect ratio of 25.

### Example 9:

In this example, components of active pharmaceutical ingredient and excipients for the preparation of an implant were: 40% (W/W) of octreotide acetate and 60% (W/W) of polylactic acid (PLA). The polylactic acid (PLA) had an Mw of 20 kDa, an intrinsic viscosity of 0.16 dL/g, and an alkyl ester group as the end group.
1) 12.0 g of octreotide acetate and 18 g of polylactic acid (PLA) were mixed, frozen ground at -40°C, the moisture content being controlled to 2.0%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 90°C, screw speed at 160 RPM, and outlet pressure below 40 Bar) and then pelletized to obtain a cylindrical implant with a diameter of 1.2 mm.
3) The implant obtained in step 2) was coated. A coating solution was 10% polylactic acid (PLA had an Mw of 20 kDa and a carboxyl group as the end group), and an organic solvent was dichloromethane. The coated implant was vacuum-dried at 25°C for 24 hours to obtain an implant with a diameter of 1.2 mm and an aspect ratio of 20.

### Example 10:

In this example, components of active pharmaceutical ingredient and excipients for the preparation of an implant were: 45% (W/W) of goserelin acetate and 55% (W/W) of PLGA. The PLGA had an Mw of 30 kDa, an intrinsic viscosity of 0.22 dL/g, and an alkyl ester group as the end group.
1) 13.5 g of goserelin acetate and 16.5 g of PLGA were mixed, frozen ground at -40°C, the moisture content being controlled to 1.0%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 95°C, screw speed at 150 RPM, and outlet pressure below 60 Bar) and then pelletized to obtain a cylindrical implant with a diameter of 1.5 mm.
3) The implant obtained in step 2) was coated. A coating solution was 15% polylactic acid (PLA had an Mw of 120 kDa and an alkyl ester group as the end group), and an organic solvent was ethyl acetate. The coated implant was vacuum-dried at 25°C for 48 hours to obtain an implant with a diameter of 1.5 mm and an aspect ratio of 23.

### Example 11:

In this example, components of active pharmaceutical ingredient and excipients for the preparation of an implant were: 40% (W/W) of exenatide and 60% (W/W) of PLGA. The PLGA had an Mw of 25 kDa, an intrinsic viscosity of 0.24 dL/g, a molar ratio of 75: 25, and an alkyl ester group as the end group.
1) 12.0 g of exenatide and 18.0 g of PLGA were mixed, frozen ground at -30°C, the moisture content being controlled to 1.5%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 100°C, screw speed at 100 RPM, and outlet pressure below 20 Bar) and then pelletized to obtain a cylindrical implant with a diameter of 1.5 mm.
3) The implant obtained in step 2) was coated. A coating solution was 10% polylactic acid (PLA had an Mw of 120 kDa and an alkyl ester group as the end group), and an organic solvent was dichloromethane. The coated implant was vacuum-dried at 25°C for 48 hours to obtain an implant with a diameter of 1.5 mm and an aspect ratio of 21.

### Example 12

In this example, components of active pharmaceutical ingredient and excipients for the preparation of an implant were: 40% (W/W) of octreotide acetate and 60% (W/W) of polylactic acid (PLA). The polylactic acid (PLA) had an Mw of 8 kDa, an intrinsic viscosity of 0.10 dL/g, and an alkyl ester group as the end group. The preparation method of an implant in this example was the same as in example 9.

### Example 13

In this example, components of active pharmaceutical ingredient and excipients for the preparation of an implant were: 40% (W/W) of octreotide acetate and 60% (W/W) of polylactic acid (PLA). The polylactic acid (PLA) had an Mw of 130 kDa, an intrinsic viscosity of 1.48 dL/g, and an alkyl ester group as the end group. The preparation method of an implant in this example was the same as in example 9.

### Comparative example 1

In this comparative example, components of active pharmaceutical ingredient and excipients for the preparation of an implant were: 30% (W/W) of varenicline tartrate and 70% (W/W) of polylactic acid (PLA). The polylactic acid (PLA) had an Mw of 20 kDa, an intrinsic viscosity of 0.20 dL/g, and an alkyl ester group as the end group.
1) 9.0 g of varenicline tartrate and 21.0 g of polylactic acid (PLA) were mixed, frozen ground at -40°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 95°C, screw speed at 100 RPM, and outlet pressure below 40 Bar) and then pelletized to obtain a cylindrical implant with a diameter of 1.6 mm.

### Comparative example 2:

In this comparative example, components of active pharmaceutical ingredient and excipients for the preparation of an implant were: 50% (W/W) of buprenorphine hydrochloride and 50% (W/W) of PLGA. The PLGA had an Mw of 100 kDa, an intrinsic viscosity of 0.82 dL/g, a molar ratio of components of 75: 25, and a carboxyl group as the end group.
1) 10.0 g of buprenorphine hydrochloride and 10.0 g of PLGA were mixed, frozen ground at -60°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 125°C, screw speed at 1500 RPM, and outlet pressure below 50 Bar) and then pelletized to obtain a cylindrical implant with a diameter of 3.0 mm.

### Comparative example 3:

In this comparative example, components of active pharmaceutical ingredient and excipients for the preparation of an implant were: 60% (W/W) of donepezil and 40% (W/W) of PLGA. The PLGA had an Mw of 40 kDa, an intrinsic viscosity of 0.32 dL/g, a molar ratio of 85: 15, and a carboxyl group as the end group.
1) 24.0 g of donepezil and 16.0 g of PLGA were mixed, frozen ground at -30°C, and sieved to acquire particles of not more than 20 mesh.
2) The mixture of active pharmaceutical ingredient and excipients obtained in step 1) was placed in a hot melt extruder for melt extrusion (with the mixing and melting zone temperature set at 120°C, screw speed at 120 RPM, and outlet pressure below 30 Bar) and then pelletized to obtain a cylindrical implant with a diameter of 2.0 mm.

### Comparative example 4:

In this comparative example, components of active pharmaceutical ingredient and excipients for the preparation of an implant were: 65% (W/W) of triamcinolone acetonide hydrochloride and 35% (W/W) of PLA-PEG-PLA. PLA-PEG-PLA had an Mw of 12 kDa, a mass percentage of PEG of 5%, and an intrinsic viscosity of 0.12 dL/g.
1) 26.0 g of triamcinolone acetonide hydrochloride and 14.0 g of PLA-PEG-PLA were mixed, frozen ground at -40°C, the moisture content being controlled to 5.0%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 90°C, screw speed at 80 RPM, and outlet pressure below 30 Bar) and then pelletized to obtain a cylindrical implant with a diameter of 2.5 mm.

### Comparative example 5:

In this comparative example, components of active pharmaceutical ingredient and excipients for the preparation of an implant were: 40% (W/W) of octreotide acetate and 60% (W/W) of polylactic acid (PLA). The polylactic acid (PLA) had an Mw of 20 kDa, an intrinsic viscosity of 0.16 dL/g, and an alkyl ester group as the end group.
1) 12.0 g of octreotide acetate and 18 g of polylactic acid (PLA) were mixed, frozen ground at -40°C, the moisture content being controlled to 2.0%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 90°C, screw speed at 160 RPM, and outlet pressure below 40 Bar) and then pelletized to obtain a cylindrical implant with a diameter of 1.2 mm.

### Comparative example 6:

In this comparative example, components of active pharmaceutical ingredient and excipients for the preparation of an implant were: 45% (W/W) of goserelin acetate and 55% (W/W) of PLGA. The PLGA had an Mw of 30 kDa, an intrinsic viscosity of 0.22 dL/g, and an alkyl ester group as the end group.
1) 13.5 g of goserelin acetate and 16.5 g of PLGA were mixed, frozen ground at -40°C, the moisture content being controlled to 1.0%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 95°C, screw speed at 150 RPM, and outlet pressure below 60 Bar) and then pelletized to obtain a cylindrical implant with a diameter of 1.5 mm.

### Comparative example 7:

In this comparative example, components of active pharmaceutical ingredient and excipients for the preparation of an implant were: 40% (W/W) of exenatide and 60% (W/W) of PLGA. The PLGA had an Mw of 25 kDa, an intrinsic viscosity of 0.24 dL/g, a molar ratio of 75: 25, and an alkyl ester group as the end group.
1) 12.0 g of exenatide and 18.0 g of PLGA were mixed, frozen ground at -30°C, the moisture content being controlled to 1.5%, and sieved to acquire particles of not more than 40 mesh.
2) The mixture of active pharmaceutical ingredient and excipients obtained in step 1) was placed in a hot melt extruder for melt extrusion (with the mixing and melting zone temperature set at 100°C, screw speed at 100 RPM, and outlet pressure below 20 Bar) and then pelletized to obtain a cylindrical implant with a diameter of 1.5 mm.

### Comparative example 8

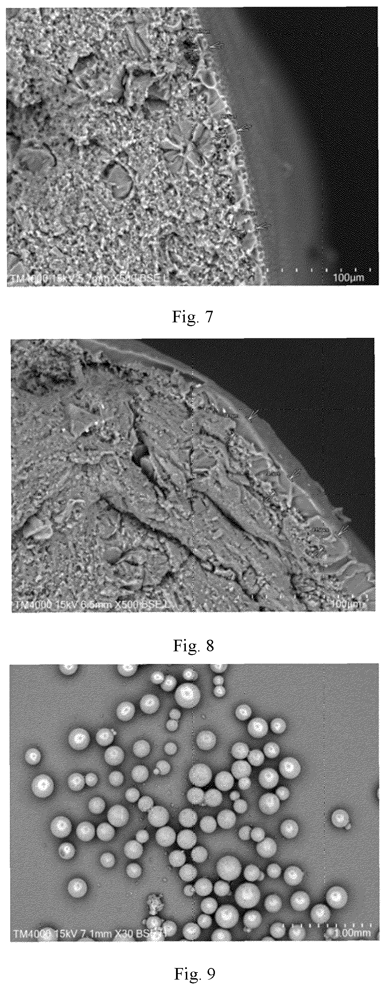

Preparation of varenicline tartrate microspheres was as follows:
1) Preparation of external aqueous phase: 50.00 g of polyvinyl alcohol (PVA, 24/88) and 400.00 g of sodium chloride were weighed, put into 5000 ml of water, continuously stirred to fully dissolve, and after the temperature had dropped to room temperature, filtered using a 100-mesh screen as the external aqueous phase.
2) Preparation of internal aqueous phase: 6.0 g of varenicline tartrate was weighed and dissolved in 24 ml of water.
3) Preparation of oil phase: the internal aqueous phase and 14.0 g of polylactic acid (PLA, intrinsic viscosity 0.20 dL/g) were added to 80.00 g of dichloromethane and vortex shaken to fully dissolve as the oil phase.
4) Emulsification: the external aqueous phase was injected into a high-shear homogenizer cavity (2000 RPM) using a peristaltic pump (2000 ml/min), and then the oil phase was injected into the high-shear homogenizer cavity using an infusion pump at 50 ml/min to form a single emulsion under high shear. The emulsion was stirred with a magnetic stirrer (500 RPM), heated to 38°C, and stirred for a further 20 hours. Dichloromethane was evaporated, and an ice bath was used for 1 h. The emulsion was then filtered, and the filter residue was acquired to obtain microspheres.
5) Freeze drying under vacuum, or other forms of drying, was performed to remove the residual solvent and moisture until the moisture content <2.1%. The particles were sieved through a sieve to obtain the varenicline tartrate microspheres (see Fig. 9 for details).

### Comparative example 9

Components of active pharmaceutical ingredient and excipients for the preparation of an implant in this comparative example were the same as those in example 4, and the preparation method was the same as that in example 4 except that the concentration of a coating solution was 3%.

### Comparative example 10

Components of active pharmaceutical ingredient and excipients for the preparation of an implant in this comparative example were the same as those in example 4, and the preparation method was the same as that in example 4 except that the concentration of a coating solution was 35%.

### Comparative example 11:

In this comparative example, components of active pharmaceutical ingredient and excipients for the preparation of a tablet were: 40% (W/W) of octreotide acetate and 60% (W/W) of polylactic acid (PLA). The polylactic acid (PLA) had an Mw of 20 kDa, an intrinsic viscosity of 0.16 dL/g, and an alkyl ester group as the end group.
1) 12.0 g of octreotide acetate and 18 g of polylactic acid (PLA) were mixed, frozen ground at -40°C, the moisture content being controlled to 2.0%, and sieved to acquire particles of not more than 40 mesh.
2) The mixture of active pharmaceutical ingredient and excipients obtained in step 1) was pressed into tablets with a sieving film coating diameter of 6 mm and a tablet weight of about 75 mg.
3) The tablet obtained in step 2) was coated. A coating solution was 10% polylactic acid (PLA had an Mw of 20 kDa and a carboxyl group as the end group), and an organic solvent was dichloromethane. The coated tablet was vacuum-dried at 25°C for 24 hours to obtain an implant with a diameter of 1.2 mm and an aspect ratio of 20.

### 1. Determination of drug loading capacity of implant

Six portions of each of the samples prepared in examples 1-13 and comparative examples 1-11 were taken, and their contents and related substances were examined by high performance liquid chromatography, which is conventional in the art. The drug loading capacity was calculated as follows: 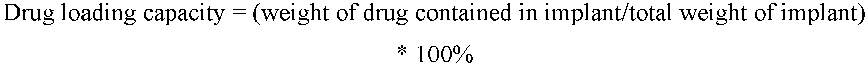 Drug loading capacity = (weight of drug contained in implant/total weight of implant) * 100%

**Table 1 Drug loading capacity and total impurities of implant**

| Sample number | Drug loading capacity (%) | Total impurities (%) |
|---|---|---|
| Example 1 | 29.86 | 0.12 |
| Example 2 | 29.95 | 0.14 |
| Example 3 | 29.96 | 0.10 |
| Example 4 | 49.25 | 0.16 |
| Example 5 | 49.32 | 0.18 |
| Example 6 | 49.16 | 0.13 |
| Example 7 | 59.83 | 0.20 |
| Example 8 | 64.92 | 0.13 |
| Example 9 | 38.97 | 0.07 |
| Example 10 | 39.05 | 0.15 |
| Example 11 | 44.17 | 0.18 |
| Example 12 | 39.05 | 0.06 |
| Example 13 | 39.74 | 0.09 |
| Comparative example 1 | 29.86 | 0.14 |
| Comparative example 2 | 50.06 | 0.18 |
| Comparative example 3 | 59.76 | 0.21 |
| Comparative example 4 | 64.93 | 0.13 |
| Comparative example 5 | 40.24 | 0.17 |
| Comparative example 6 | 44.56 | 0.11 |
| Comparative example 7 | 39.46 | 0.16 |
| Comparative example 8 | 26.52 | 0.24 |
| Comparative example 9 | 49.36 | 0.15 |
| Comparative example 10 | 47.76 | 0.16 |
| Comparative example 11 | 39.82 | 0.13 |

It can be seen from the test results of the drug loading capacity and related substances of the samples in Table 1 that the actual drug loading capacity of the implant is very close to the theoretical drug loading capacity and higher than that of microspheres. That is, the HME process has almost no loss of active pharmaceutical ingredient and excipients. There was no change in the content after thermal passivation. However, after the coating process, because the coating layer has a certain thickness, the drug loading capacity is reduced to different degrees, which is determined by the coating composition and coating thickness of each variety. In addition, the total impurities of the samples prepared in examples and comparative examples are low, and there is no significant difference, indicating that the thermal passivation or coating process has no effect on the impurities of water-soluble drug implants.

### 2. In vitro release degree test

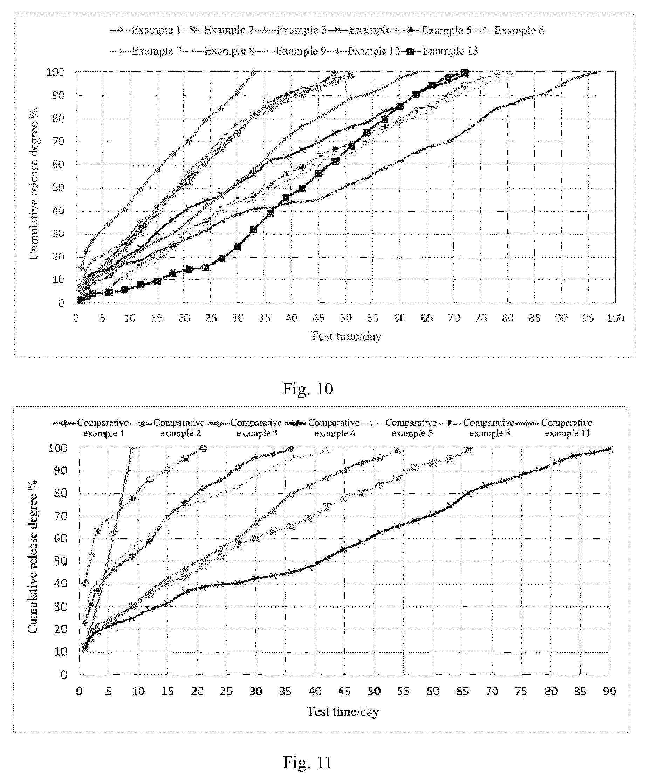

The determination method was as follows: six portions (30 mg) of each of the implants prepared in examples 1-13, comparative examples 1-5, and comparative examples 8-11 were weighed and placed in a 100 ml Erlenmeyer flask. The release medium was 50 ml of phosphate buffer, pH 7.4, and the implants were examined using a 37 ± 0.5°C constant temperature water bath oscillator at a rotation speed of 50 RPM. Samples were taken at preset time points (after taking samples on day 1 and day 3 and replacing the medium, samples were taken and the medium was replaced every 3 or 4 days until taking samples on day 90, ensuring that the whole release process maintained sink conditions), and the sample contents were detected using high performance liquid chromatograph. The average cumulative release curve (see Figs. 10-11 for details) and the average daily release rate (see Table 2 for details) were calculated.

**Table 2 In vitro average cumulative release degree (%) of examples**

| Sampling time point/d | Example 1 | Example 2 | Example 3 | Example 4 | Example 5 | Example 6 | Example 7 | Example 8 | Example 9 | Example 12 | Example 13 |
|---|---|---|---|---|---|---|---|---|---|---|---|
| 1 | 4.06 | 3.88 | 3.96 | 5.88 | 1.75 | 0.86 | 6.45 | 4.74 | 8.12 | 15.24 | 1.03 |
| 2 | 8.35 | 6.89 | 7.87 | 10.47 | 2.63 | 2.12 | 8.87 | 6.47 | 14.51 | 22.73 | 2.57 |
| 3 | 11.67 | 10.57 | 11.02 | 12.68 | 3.74 | 3.54 | 10.34 | 8.76 | 18.35 | 26.63 | 3.88 |
| 6 | 18.48 | 16.98 | 17.53 | 14.63 | 6.08 | 5.14 | 13.42 | 11.48 | 22.47 | 34.38 | 4.56 |
| 9 | 25.76 | 23.67 | 23.47 | 19.74 | 12.07 | 10.87 | 17.86 | 16.77 | 26.73 | 40.76 | 5.45 |
| 12 | 32.57 | 30.47 | 31.52 | 23.78 | 16.35 | 14.68 | 22.75 | 18.45 | 35.67 | 49.52 | 7.76 |
| 15 | 41.73 | 39.76 | 38.86 | 30.45 | 20.49 | 17.98 | 26.83 | 22.54 | 40.15 | 57.47 | 9.58 |
| 18 | 48.47 | 47.48 | 47.85 | 36.41 | 25.36 | 23.87 | 30.17 | 24.78 | 48.08 | 64.56 | 12.74 |
| 21 | 54.58 | 52.98 | 52.47 | 41.25 | 31.79 | 28.43 | 35.73 | 28.32 | 57.36 | 70.17 | 14.45 |
| 24 | 61.42 | 61.75 | 60.42 | 44.35 | 35.47 | 32.75 | 41.75 | 31.33 | 63.47 | 79.45 | 15.53 |
| 27 | 68.36 | 67.54 | 66.85 | 46.75 | 41.23 | 40.16 | 46.76 | 35.57 | 71.58 | 84.66 | 19.47 |
| 30 | 74.08 | 73.68 | 73.12 | 51.63 | 44.52 | 43.57 | 52.47 | 38.45 | 77.84 | 91.58 | 24.45 |
| 33 | 81.14 | 81.47 | 81.13 | 55.65 | 46.56 | 44.35 | 57.63 | 40.85 | 80.84 | 99.84 | 31.76 |
| 36 | 86.78 | 85.86 | 85.63 | 61.47 | 51.32 | 48.73 | 64.76 | 41.35 | 83.69 | - | 38.98 |
| 39 | 90.34 | 88.76 | 88.25 | 63.33 | 55.98 | 52.34 | 71.45 | 43.25 | 87.78 | - | 45.76 |
| 42 | 92.65 | 91.69 | 90.34 | 66.34 | 59.12 | 55.73 | 76.53 | 44.05 | 91.18 | - | 49.78 |
| 45 | 94.74 | 93.54 | 93.65 | 69.56 | 63.55 | 60.18 | 80.43 | 45.08 | 94.64 | - | 56.36 |
| 48 | 99.59 | 95.76 | 96.72 | 73.54 | 66.71 | 64.73 | 84.47 | 48.23 | 97.34 | - | 61.65 |
| 51 | - | 99.74 | 98.49 | 76.53 | 68.98 | 64.63 | 88.76 | 51.75 | 99.08 | - | 67.84 |
| 54 | - | - | - | 78.49 | 73.24 | 69.47 | 90.35 | 54.35 | - | - | 74.18 |
| 57 | - | - | - | 82.96 | 76.43 | 74.18 | 93.45 | 58.37 | - | - | 79.85 |
| 60 | - | - | - | 85.56 | 79.17 | 77.84 | 97.43 | 61.48 | - | - | 85.41 |
| 63 | - | - | - | 90.46 | 83.56 | 80.43 | 99.75 | 65.26 | - | - | 90.43 |
| 66 | - | - | - | 93.76 | 86.09 | 83.64 | - | 67.88 | - | - | 94.53 |
| 69 | - | - | - | 95.85 | 90.15 | 88.09 | - | 70.06 | - | - | 97.86 |
| 72 | - | - | - | 99.08 | 94.65 | 91.34 | - | 74.24 | - | - | 99.79 |
| 75 | - | - | - | - | 96.84 | 93.73 | - | 79.27 | - | - | - |
| 78 | - | - | - | - | 99.64 | 96.73 | - | 84.36 | - | - | - |
| 81 | - | - | - | - | - | 99.45 | - | 86.47 | - | - | - |
| 84 | - | - | - | - | - | - | - | 89.06 | - | - | - |
| 87 | - | - | - | - | - | - | - | 90.9 | - | - | - |
| 90 | - | - | - | - | - | - | - | 94.68 | - | - | - |
| 93 | - | - | - | - | - | - | - | 97.86 | - | - | - |
| 96 | - | - | - | - | - | - | - | 99.63 | - | - | - |

**Table 3 In vitro average cumulative release degree (%) of comparative examples**

| Sampling time point/d | Comparati ve example 1 | Compara tive example 2 | Compara tive example 3 | Compara tive example 4 | Compara tive example 5 | Compara tive example 8 | Compara tive example 9 | Compara tive example 10 | Compara tive example 11 |
|---|---|---|---|---|---|---|---|---|---|
| 1 | 22.82 | 12.54 | 12.23 | 11.47 | 25.84 | 40.47 | 11.74 | 0.24 | 13.24 |
| 2 | 30.60 | 16.35 | 16.54 | 16.78 | 36.67 | 52.51 | 16.45 | 0.42 | 20.75 |
| 3 | 36.76 | 19.49 | 21.78 | 18.82 | 40.15 | 63.57 | 20.54 | 0.98 | 29.74 |
| 6 | 46.55 | 24.36 | 25.55 | 22.47 | 48.95 | 70.59 | 24.78 | 1.87 | 63.23 |
| 9 | 52.12 | 29.79 | 30.58 | 24.89 | 56.36 | 77.84 | 30.48 | 3.17 | 99.76 |
| 12 | 58.97 | 35.41 | 36.87 | 28.74 | 61.47 | 86.32 | 35.78 | 4.78 | - |
| 15 | 69.78 | 40.22 | 42.35 | 31.45 | 68.58 | 90.45 | 40.16 | 5.68 | - |
| 18 | 75.94 | 43.23 | 46.87 | 36.25 | 73.84 | 95.68 | 44.78 | 6.43 | - |
| 21 | 82.22 | 47.56 | 51.12 | 38.45 | 76.84 | 99.87 | 48.76 | 8.42 | - |
| 24 | 85.74 | 52.31 | 55.69 | 39.85 | 79.99 | - | 52.39 | 9.75 | - |
| 27 | 91.39 | 56.74 | 60.17 | 40.35 | 82.69 | - | 57.42 | 10.75 | - |
| 30 | 95.73 | 60.12 | 66.89 | 42.25 | 87.78 | - | 60.45 | 11.74 | - |
| 33 | 97.36 | 63.33 | 72.58 | 43.58 | 91.12 | - | 63.47 | 13.47 | - |
| 36 | 99.68 | 65.58 | 79.56 | 45.08 | 95.64 | - | 66.86 | 15.73 | - |
| 39 | - | 68.98 | 83.33 | 47.23 | 96.34 | - | 68.79 | 17.84 | - |
| 42 | - | 74.12 | 86.95 | 51.21 | 99.08 | - | 73.14 | 19.43 | - |
| 45 | - | 77.86 | 90.47 | 55.35 | - | - | 78.43 | 20.07 | - |
| 48 | - | 80.45 | 93.68 | 58.36 | - | - | 80.14 | 21.47 | - |
| 51 | - | 83.81 | 95.77 | 62.48 | - | - | 83.64 | 22.46 | - |
| 54 | - | 86.77 | 99.05 | 65.36 | - | - | 87.43 | 23.34 | - |
| 57 | - | 91.66 | - | 67.78 | - | - | 90.48 | 24.73 | - |
| 60 | - | 93.63 | - | 70.68 | - | - | 93.65 | 26.47 | - |
| 63 | - | 95.25 | - | 74.44 | - | - | 97.76 | 27.74 | - |
| 66 | - | 98.89 | - | 79.87 | - | - | 99.08 | 29.43 | - |
| 69 | - | - | - | 83.36 | - | - | - | 31.18 | - |
| 72 | - | - | - | 85.47 | - | - | - | 32.46 | - |
| 75 | - | - | - | 88.06 | - | - | - | 33.13 | - |
| 78 | - | - | - | 90.31 | - | - | - | 35.14 | - |
| 81 | - | - | - | 93.68 | - | - | - | 37.07 | - |
| 84 | - | - | - | 96.57 | - | - | - | 38.76 | - |
| 87 | - | - | - | 97.86 | - | - | - | 40.45 | - |
| 90 | - | - | - | 99.63 | - | - | - | 41.53 | - |
| 93 | - | - | - | - | - | - | - | - | - |

By analyzing the in vitro release degree test results of the samples prepared in examples 1-3 and comparative example 1, it can be seen that thermal passivation can significantly control the burst release phenomenon of the water-soluble drug at the initial stage so that the drug is released in zero order in the whole sustained release cycle, thereby avoiding the adverse reactions of patients caused by the burst release of the drug. The thermal passivation temperature should be selected according to the HME temperature required for the implant and is usually not lower than the HME temperature, but should not be too high to prevent secondary melting and severe deformation of the implant. The required time for thermal passivation time need to be comprehensively selected in conjunction with the thermal passivation temperature. That is, the required time will vary under different thermal passivation temperatures, depending on the control of the release degree of the final product.

By analyzing the in vitro release degree test results of the samples prepared in examples 4-6 and comparative example 2, it can be seen that coating the implant can significantly reduce the release speed of the water-soluble drug at the initial stage so that the drug is released in zero order in the whole sustained release cycle, thereby avoiding the adverse reactions of patients caused by the burst release of the drug. The composition of the coating solution may depend on the required controlled release speed. Generally, biodegradable polymers with low molecular weight or intrinsic viscosity will release drugs more quickly than those with high molecular weight. Linear biodegradable polymers will release drugs more quickly than multi-branched star-shaped ones. Hydrophobic end-capped biodegradable polymers will release drugs more quickly than hydrophilic end-capped ones. PEG-modified biodegradable polymers will release drugs more quickly than unmodified ones, and low-concentration polymers will release drugs more quickly than high-concentration polymers. The in vitro release degrees of comparative example 9 and comparative example 10 show that if the concentration of the coating solution is too low, there will be no obvious controlled release effect. If the concentration of the coating solution is too high, the drug release will be seriously blocked, resulting in a delayed release phenomenon, which is not suitable for water-soluble drug varieties requiring rapid action.

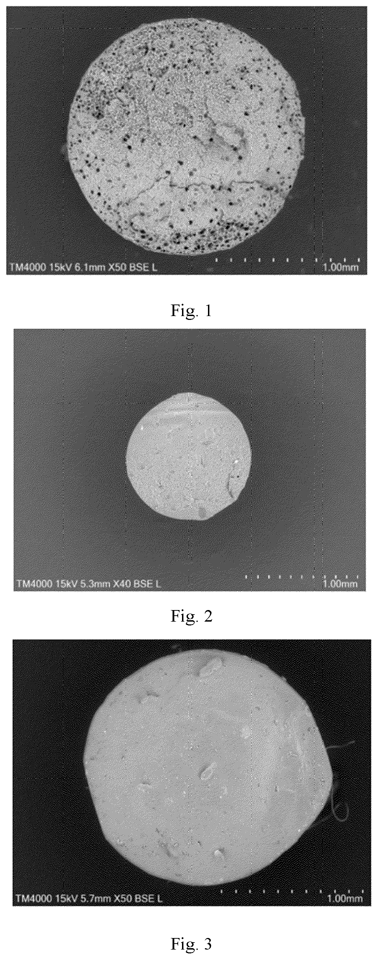

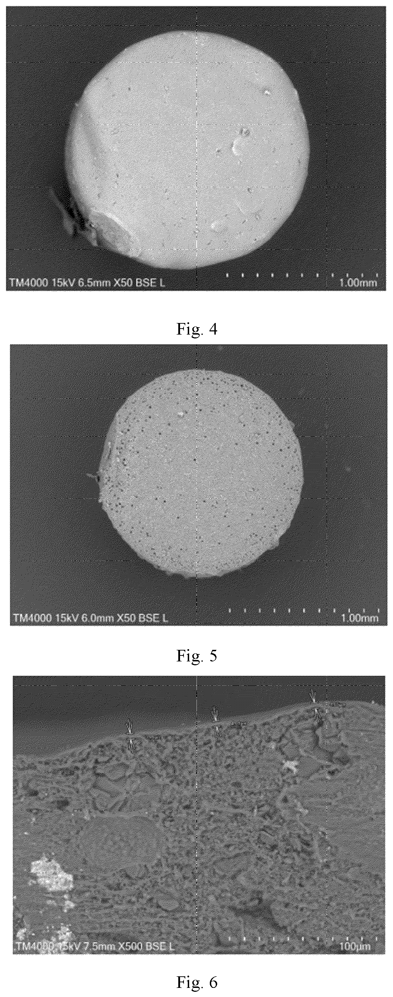

By analyzing the in vitro release degrees of examples 1-3, comparative example 1, and comparative example 8, it was found that varenicline tartrate microspheres had severe burst release, and the drug was completely released in about 20 days. The implant prepared by the HME process could reduce the burst release to some extent, but the release amount in the first day was still large (> 20%). However, a dense controlled-release film coating was formed on the outer surface of the implant through the thermal passivation or coating process, and the pores for drug dissolution/diffusion were significantly reduced (see Figs. 1-8), thereby avoiding burst release. The initial release speed may be controlled within the expected range, which is very suitable for the development of BCS class 1 and BCS class 3 drugs into long-acting sustained and controlled release implants.

In example 12, PLA having an Mw of 8 kDa and an intrinsic viscosity of 0.10 dL/g was adopted, and the drug release rate was faster than that in example 9. The reason is that the biodegradable polymer has a low molecular weight and a small intrinsic viscosity and is easily hydrolyzed or degraded by itself, resulting in a faster dissolution/erosion of octreotide acetate. However, the drug release rate in example 12 was slower than that in comparative example 5, indicating that coating could control the early burst release to some extent.

In example 13, PLA having an Mw of 130 kDa and an intrinsic viscosity of 1.48 dL/g was adopted. Compared with example 9 and comparative Example 5, the drug release at the early stage was significantly slowed down, and the cumulative release amount of the drug during 15 days was about 10%. The reason is that PLA has an Mw of more than 12 kDa and an intrinsic viscosity of more than 1.2 dL/g, and many hydrophobic groups make it difficult for water to enter the pores of the matrix so that the drug is firmly bound in PLA, resulting in a relatively delayed release phenomenon.

The HME and coating processes were adopted in example 9, and the coating process was not adopted in comparative example 5. By analyzing the in vitro release degrees of comparative example 5 and example 9, it was found that the early release in comparative example 5 was controlled to a certain extent. With the extension of release time, the drug release was accelerated, and the release cycle was significantly less than that of the sample of example 9. This indicates that the coating process can form a film coating-controlled sustained release effect. This is because as the outer coating film coating of the implant degrades, the porosity of the film coating increases, and there is less resistance to drug release from the pores of the film coating, resulting in an increased release rate. The HME and coating processes were adopted in example 9, and the tableting and coating processes were adopted in comparative example 11. By analyzing the in vitro release degrees of comparative example 11 and example 9, it was found that the matrix-type sustained release structure could be formed by the HME, and the release cycle of the drug core prepared by the HME was longer than that of tablet core obtained by tableting. Therefore, in order to prolong the drug sustained release cycle, the HME and coating/passivation processes were adopted to achieve the dual sustained and controlled release effect, thereby making the drug concentration in the blood more stable and prolonging the drug release cycle.

Abbreviations and English meanings in the present disclosure are set forth in Table 4.

**Table 4 Abbreviations and English meanings**

| Abbreviations and English | Meanings |
|---|---|
| BCS | Biopharmaceutics classification system |
| PLA | Polylactide |
| PLGA | poly(lactide-co-glycolide) |
| PCL | Polycaprolactone |
| PEG | Polyethylene glycol |
| Mw | Weight-average molecular weight |
| SEM | Scanning electron microscope |

Finally, it should be noted that the above-mentioned contents are merely illustrative of the technical solutions of the present disclosure and do not limit the scope of the present disclosure.

## Claims

1. A preparation method of an implant, comprising the steps of:
(1) mixing an active drug with a biodegradable polymer, freeze-grinding, drying, and sieving, resulting in a mixture;
(2) performing melt extrusion on the mixture obtained in step (1), then cooling, shaping, and pelletizing to obtain shaped solids; and
(3) performing thermal passivation or coating on the shaped solids obtained in step (2), then drying to obtain an implant;
wherein the thermal passivation in step (3) is performed at a temperature of 90-140°C for 5-30 min.

2. The preparation method according to claim 1, wherein a weight ratio of the active drug to the biodegradable polymer is 30-65: 35-70, preferably 35-60: 40-65.

3. The preparation method according to claim 1, wherein the active drug comprises varenicline, buprenorphine, donepezil, triamcinolone acetonide, octreotide, leuprorelin, goserelin, exenatide, or pharmaceutically acceptable salts thereof.

4. The preparation method according to claim 1, wherein the biodegradable polymer comprises one or more of polylactide (PLA), polyglycolide(PGA), poly(lactide-co-glycolide) (PLGA), polycaprolactone (PCL), PLA-PEG, PLGA-PEG, PLGA-PEG-PLGA, PLA-PEG-PLA, and PCL-PEG-PCL, preferably PLA, PLGA, PLA-PEG, PLGA-PEG, PLGA-PEG-PLGA, and PLA-PEG-PLA, and further preferably PLA and/or PLGA.

5. The preparation method according to claim 1, wherein the freeze-grinding in step (1) is performed at a temperature of -70°C to -20°C, preferably -50°C to -25°C.

6. The preparation method according to claim 1, wherein the thermal passivation in step (3) is performed at a temperature of 95-130°C for 8-30 min.

7. The preparation method according to claim 1, wherein a coating solution used for the coating in step (3) comprises a sustained-release composition and an organic solvent; the sustained-release composition comprises one or more of PLA, PGA, PLGA, PLA-PEG, PLGA-PEG, PLGA-PEG-PLGA, PLA-PEG-PLA, and preferably PLA and/or PLGA.

8. The preparation method according to claim 7, wherein a weight proportion of the sustained-release composition in the coating solution is 3-30%, preferably 6-25%.

9. The preparation method according to claim 4 or 7, wherein each of PLA and PLGA has a weight-average molecular weight (Mw) of 7,000-150,000 Da and an intrinsic viscosity of 0.1-2.5 dL/g, and preferably an Mw of 9,000-120,000 Da and an intrinsic viscosity of 0.2-1.2 dL/g.

## Patentansprüche

1. Herstellungsverfahren für ein Implantat, die folgenden Schritte umfassend:
(1) Mischen eines Wirkstoffs mit einem biologisch abbaubaren Polymer, Gefriermahlen, Trocknen und Sieben, wodurch eine Mischung entsteht;
(2) Durchführen einer Schmelzextrusion der in Schritt (1) erhaltenen Mischung, anschließend Kühlen, Formen und Pelletieren, um geformte Feststoffe zu erhalten; und
(3) Durchführen einer thermischen Passivierung oder Beschichtung der in Schritt (2) erhaltenen geformten Feststoffe, anschließend Trocknen, um ein Implantat zu erhalten;
wobei die thermische Passivierung in Schritt (3) bei einer Temperatur von 90-140 °C für 5-30 min durchgeführt wird.

2. Herstellungsverfahren nach Anspruch 1, wobei das Gewichtsverhältnis des Wirkstoffs zum biologisch abbaubaren Polymer 30-65 : 35-70, vorzugsweise 35-60 : 40-65 beträgt.

3. Herstellungsverfahren nach Anspruch 1, wobei der Wirkstoff Vareniclin, Buprenorphin, Donepezil, Triamcinolonacetonid, Octreotid, Leuprorelin, Goserelin, Exenatid oder pharmazeutisch verträgliche Salze davon umfasst.

4. Herstellungsverfahren nach Anspruch 1, wobei das biologisch abbaubare Polymer eines oder mehrere der folgenden umfasst: Polylactid (PLA), Polyglycolid (PGA), Poly(lactid-co-glycolid) (PLGA), Polycaprolacton (PCL), PLA-PEG, PLGA-PEG, PLGA-PEG-PLGA, PLA-PEG-PLA und PCL-PEG-PCL, vorzugsweise PLA, PLGA, PLA-PEG, PLGA-PEG, PLGA-PEG-PLGA und PLA-PEG-PLA, und noch bevorzugter PLA und/oder PLGA.

5. Herstellungsverfahren nach Anspruch 1, wobei das Gefriermahlen in Schritt (1) bei einer Temperatur von -70 °C bis -20 °C, vorzugsweise von -50 °C bis -25 °C, durchgeführt wird.

6. Herstellungsverfahren nach Anspruch 1, wobei die thermische Passivierung in Schritt (3) bei einer Temperatur von 95-130 °C für 8-30 min durchgeführt wird.

7. Herstellungsverfahren nach Anspruch 1, wobei eine für die Beschichtung in Schritt (3) verwendete Beschichtungslösung eine Zusammensetzung mit verzögerter Freisetzung und ein organisches Lösungsmittel umfasst; wobei die Zusammensetzung mit verzögerter Freisetzung eines oder mehrere der folgenden umfasst: PLA, PGA, PLGA, PLA-PEG, PLGA-PEG, PLGA-PEG-PLGA, PLA-PEG-PLA, vorzugsweise PLA und/oder PLGA.

8. Herstellungsverfahren nach Anspruch 7, wobei der Gewichtsanteil der Zusammensetzung mit verzögerter Freisetzung in der Beschichtungslösung 3-30 %, vorzugsweise 6-25 % beträgt.

9. Herstellungsverfahren nach Anspruch 4 oder 7, wobei sowohl PLA als auch PLGA eine gewichtsmittlere Molmasse (Mw) von 7.000-150.000 Da und eine intrinsische Viskosität von 0,1-2,5 dL/g aufweisen, vorzugsweise eine Mw von 9.000-120.000 Da und eine intrinsische Viskosität von 0,2-1,2 dL/g.

## Revendications

1. Procédé de préparation d'un implant, comprenant les étapes consistant à :
(1) mélanger un principe actif avec un polymère biodégradable, effectuer un broyage cryogénique, sécher et tamiser, pour obtenir un mélange ;
(2) soumettre le mélange obtenu à l'étape (1) à une extrusion à l'état fondu, puis refroidir, mettre en forme et granuler pour obtenir des solides mis en forme ; et
(3) soumettre les solides mis en forme obtenus à l'étape (2) à une passivation thermique ou à un enrobage, puis sécher pour obtenir un implant ;
dans lequel la passivation thermique à l'étape (3) est effectuée à une température de 90-140 °C pendant 5-30 min.

2. Procédé de préparation selon la revendication 1, dans lequel un rapport pondéral du principe actif au polymère biodégradable est de 30-65 : 35-70, de préférence de 35-60 : 40-65.

3. Procédé de préparation selon la revendication 1, dans lequel le principe actif comprend la varénicline, la buprénorphine, le donépézil, l'acétonide de triamcinolone, l'octréotide, la leuproréline, la goséréline, l'exénatide, ou leurs sels pharmaceutiquement acceptables.

4. Procédé de préparation selon la revendication 1, dans lequel le polymère biodégradable comprend un ou plusieurs composés choisis parmi le polylactide (PLA), le polyglycolide (PGA), le poly(lactide-co-glycolide) (PLGA), la polycaprolactone (PCL), le PLA-PEG, le PLGA-PEG, le PLGA-PEG-PLGA, le PLA-PEG-PLA et le PCL-PEG-PCL, de préférence le PLA, le PLGA, le PLA-PEG, le PLGA-PEG, le PLGA-PEG-PLGA et le PLA-PEG-PLA, et de manière encore plus préférée le PLA et/ou le PLGA.

5. Procédé de préparation selon la revendication 1, dans lequel le broyage cryogénique à l'étape (1) est effectué à une température de -70 °C à -20 °C, de préférence de -50 °C à -25 °C.

6. Procédé de préparation selon la revendication 1, dans lequel la passivation thermique à l'étape (3) est effectuée à une température de 95-130 °C pendant 8-30 min.

7. Procédé de préparation selon la revendication 1, dans lequel une solution d'enrobage utilisée pour l'enrobage à l'étape (3) comprend une composition à libération prolongée et un solvant organique ; la composition à libération prolongée comprend un ou plusieurs composés choisis parmi le PLA, le PGA, le PLGA, le PLA-PEG, le PLGA-PEG, le PLGA-PEG-PLGA, le PLA-PEG-PLA, et de préférence le PLA et/ou le PLGA.

8. Procédé de préparation selon la revendication 7, dans lequel une proportion pondérale de la composition à libération prolongée dans la solution d'enrobage est de 3-30 %, de préférence de 6-25 %.

9. Procédé de préparation selon la revendication 4 ou 7, dans lequel le PLA et le PLGA présentent chacun une masse moléculaire moyenne en poids (Mw) de 7 000 à 150 000 Da et une viscosité intrinsèque de 0,1-2,5 dL/g, et de préférence une Mw de 9 000 à 120 000 Da et une viscosité intrinsèque de 0,2-1,2 dL/g.
